# EUROPEAN PATENT SPECIFICATION

(11) **EP 1 649 030 B1**
(45) Date of publication and mention of the grant of the patent: **25.06.2014**
(21) Application number: 04779304.7
(22) Date of filing: 27.07.2004
(51) Int. Cl.: C12P 1/00, C12P 7/00, C12P 7/26, C12P 1/04, C12N 9/04, C07H 21/04

(54) **METABOLICALLY ENGINEERED BACTERIAL STRAINS HAVING ENHANCED 2-KETO-D-GLUCONATE ACCUMULATION**
METABOLISCH MANIPULIERTE BAKTERIENSTÄMME MIT VERBESSERTER 2-KETO-D-GLUCONAT-ANREICHERUNG
SOUCHES BACTERIENNES TRANSFORMEES DE MANIERE METABOLIQUE PRESENTANT UNE ACCUMULATION AMELIOREE DE 2-CETO-D-GLUCONATE

(30) Priority: 30.07.2003 US 491150 P
(43) Date of publication of application: 26.04.2006
(73) Proprietor: Danisco US Inc., Palo Alto, CA 94304 (US)
(72) Inventor: DODGE, Timothy, C., Sunnyvale, CA 94010 (US); VALLE, Fernando, Burlingame, CA 94010 (US); RASHID, M., Harunur, Sunnyvale, CA 94087 (US)
(74) Representative: Kiddle, Simon John
(86) International application number: PCT/US2004/024203
(87) International publication number: WO 2005/012486

(56) References cited:
- WO-A2-02/081440
- US-B2- 6 599 722
- DATABASE EMBL [Online] EBI; Pantoea citrea 1 November 1999 (1999-11-01), PUJOL C.J.: "2-keto-gluconate dehydrogenase subunit" XP002416923 retrieved from UNIPROT accession no. Q9XCR1 Database accession no. Q9XCR1
- DATABASE EMBL [Online] EBI; PKDGB of Pantoea citrea 1 November 1999 (1999-11-01), PUJOL C.D.: "2-ketogluconate dehydrogenase subunit" XP002416924 retrieved from UNIPROT accession no. Q9XCR0_PANCI Database accession no. Q9XCR0
- DATABASE EMBL [Online] EBI; KDGC of Pantoea citrea 1 November 1999 (1999-11-01), PUJOL C.J.: "2-keto-gluconate dehydrogenase cytrochrome c subunit" XP002416925 retrieved from UNIPROT accession no. Q9XCQ9_PANCI Database accession no. Q9XCQ9
- ADACHI O ET AL: 'New development in oxidative fermentation.' APPL MICROBIOL BIOTECHNOL. vol. 60, no. 6, February 2003, pages 643 - 653, XP002351155
- PUJOL CJ ET AL: 'Genetic and Biochemical Characterization of the Pathway in Pantoea Citrea Leading to Pink Disease of Pineapple.' J BACTERIOL. vol. 182, no. 8, April 2000, pages 2230 - 2237, XP002992380
- MCINTIRE W ET AL: 'Identification of the covalently bound flavins of D-gluconate dehydrogenases from Pseudomonas aeruginosa and Pseudomonas fluorescens and of 2-keto-D-gluconate dehydrogenase from Gluconobacter melanogenus.' J BIOCHEM. vol. 231, no. 3, 01 November 1985, pages 651 - 654, XP008056963

## Description

### 1. BACKGROUND OF THE INVENTION

The present invention relates to a method of altering bacterial host cells to accumulate 2-keto-D-gluconic acid (2-KDG) by inactivating an endogenous membrane bound 2-keto-D-gluconate dehydrogenase (2-KDGDH), which prior to inactivation catalyzed the conversion of 2-KDG to 2,5-diketogluconate (2,5-DKG). The invention particularly relates to enhancing the biosynthetic accumulation of 2-KDG in *Pantoea* strains and the use of the 2-KDG so produced.

Numerous products of commercial interest, such as intermediates of L-ascorbic acid, have been produced biocatalytically in genetically engineered host cells. While in many instances, the desired end-product of the bioconversion may be L-ascorbic acid (ASA, vitamin C), there exists independent uses for the ASA intermediates and these ASA intermediates may be the desired product of bioconversion.

One process for biocatalytically converting ordinary metabolites such as glucose into ASA intermediates is shown in Figure 1. Through oxidative steps glucose is converted to 2-KDG and 2,5-DKG (USP 3,790,444). Also reference is made to a bioconversion method for the production of the ASA intermediate, 2-keto-L-gulonic acid (2-KLG), which has been disclosed by Lazarus et al. (1989, "Vitamin C: Bioconversion via a Recombinant DNA Approach", GENETICS AND MOLECULAR BIOLOGY OF INDUSTRIAL MICROORGANISMS, American Society for Microbiology, Washington D.C. Edited by C.L. Hershberger). This bioconversion of a carbon source to 2-KLG involves a variety of intermediates, and the enzymatic process is associated with co-factor dependent 2,5-DKG reductase activity (DKGR). Additionally, recombinant DNA techniques have been used to bioconvert glucose to 2-KLG in *Erwinia herbicola* in a single fermentative step (Anderson, S. et al., (1985) Science 230:144 - 149).

In this application, the inventors are concerned with increasing the accumulation of the ASA intermediate, 2-keto-D-gluconic acid (2-KDG) in a bacterial host. Preferably, a bacterial host cell capable of converting 2-KDG to 2,5-DKG by the activity of an endogenous membrane bound 2-keto-D-gluconate dehydrogenase (2-KDGDH) enzyme is altered in a manner to inactivate the 2-KDGDH enzyme.

The 2-KDG, which is accumulated in the altered bacterial cells encompassed by the invention may then be used in various industrial and commercial applications. For example, 2-KDG may be used for the solubilization of phosphates or as an intermediate in the synthesis of herbicidal compounds, such as those disclosed in USP 3,981,860. Additionally, the 2-KDG may be further converted to desired end-products, such as erythorbic acid. Erythorbic acid is a stereoisomer of ASA and is also known as D-araboascorbic acid. This compound may be chemically synthesized according to the method of Reichstein and Grussner (1934) Helv. Chim. Acta 17:311-328. Erythorbic acid is a well-known food additive, preservative and antioxidant and is used in industrial and specialty chemical applications.

WO 02/081440 discloses processes for the production of ascorbic acid intermediates in *Pantoea* cells such that the intermediates accumulate in the cells through the inactivation of the glucokinase or gluconokinase genes.

Pujol et al. (J. Bacteriology, 182(8): 2230-2237, 2000) describes the genetic and biochemical characterization of the pathway in *Pantoea citrea* that leads to pink disease in pineapple plants.

### 2. SUMMARY OF THE INVENTION

The present invention relates to a method of accumulating 2-KDG in a bacterial host cell by culturing an altered host cell in the presence of a carbon source, such as glucose or other ordinary microbial metabolite. More specifically, by inactivating an endogenous gene which encodes a membrane bound 2-keto-D-gluconate dehydrogenase (2-KDGDH) enzyme, 2-KDG is not converted to the intermediate 2,5-DKG, and 2-KDG accumulates in the altered host cell. The 2-KDG can then be isolated from the altered host cell and used in various commercial applications or further be used to produce other desired compounds, such as erythorbic acid.

Accordingly, in one aspect, the present invention provides a method for increasing the accumulation of 2-keto-D-gluconic acid (2- KDG) in a *Pantoea* host cell comprising:
a) inactivating in a *Pantoea* host cell, which is capable of producing 2,5-diketogluconate (2,5-DKG) from the enzymatic conversion of 2-KDG in the presence of a carbon source, at least one endogenous gene necessary for 2-keto-D-gluconate dehydrogenase (2-KDGDH) activity, wherein the at least one endogenous gene encodes (i) a dehydrogenase protein having at least 95% sequence identity to SEQ ID NO: 4, (ii) a cytochrome C protein having at least 95% sequence identity to SEQ ID NO 6, or (iii) a protein having at least 95% sequence identity to SEQ ID NO: 2, and wherein inactivating the at least one endogenous gene comprises inserting by homologous recombination an inactivation cassette comprising an antibiotic resistance marker to obtain an altered *Pantoea* cell; and
b) culturing the altered *Pantoea* cell under suitable culture conditions to produce 2-KDG to allow 2-KDG to accumulate in the *Pantoea* host cell.

### 3. BRIEF DESCRIPTION OF THE DRAWINGS

Figure 1 depicts the oxidative pathway for the production of ascorbic acid (ASA). E1 stands for glucose dehydrogenase (GDH); E2 stands for gluconic acid dehydrogenase (GADH); E3 stands for 2-keto-D-gluconic acid dehydrogenase (2-KDGDH or 2-KGDH); and E4 stands for 2,5-diketo-D-gluconic acid reductase (2,5 DKGR).

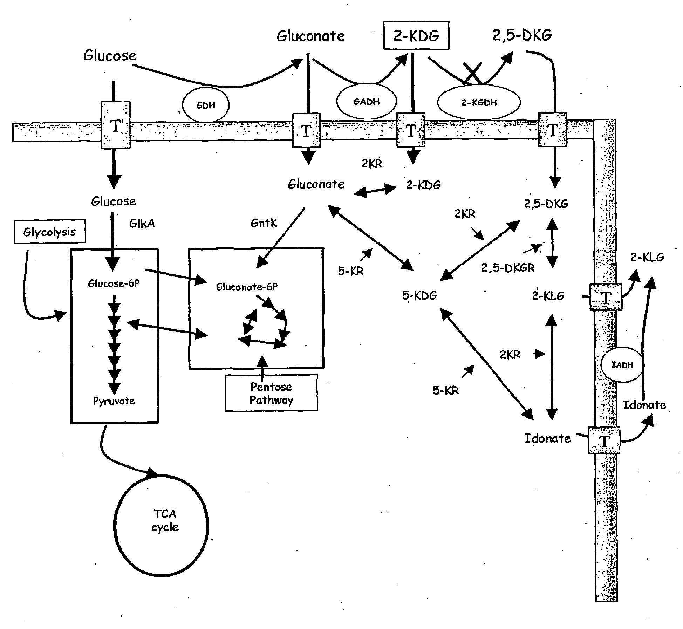

Figure 2 provides a schematic representation of some of the metabolic routes involved in glucose assimilation in a bacterial host cell such as *Pantoea citrea.* The diagram shows the most common connections between the glycolytic, pentose and tricarboxylic (TCA) pathways. The following abbreviations have been used in the figure and are applied throughout the disclosure: glucose dehydrogenase = (GDH); gluconic acid dehydrogenase = (GADH); 2-keto-D-gluconate = (2-KDG); 2-keto-D-gluconic acid dehydrogenase = (2-KDGDH or 2-KGDH); 2,5-diketogluconate = (2,5-DKG); 2,5-diketo-D-gluconic acid reductase = (2, 5 DKGR); 2-keto-L-gulonic acid = (2-KLG); 2-ketoreductase = (2KR); 5-ketoreductase = (5KR); 5-keto-D-gluconate =(5-KDG); idonate dehydrogenase = (IADH); glucokinase = (GlkA) and gluconokinase = (GntK). The enzymatic step affected by the inactivation of 2-KDGDH according to the present invention is indicated by a "X". Boxes labeled with a "T" represent putative transporters.
Figure 3A depicts a nucleic acid sequence (SEQ ID NO. 1) encoding an amino acid sequence of subunit A of a *Pantoea citrea* 2-KDGDH enzyme.
Figure 3B depicts the amino acid sequence of subunit A (SEQ ID NO. 2) encoded by SEQ ID NO: 1.
Figure 4 depicts a nucleic acid sequence (SEQ ID NO. 3) encoding an amino acid sequence of subunit B of a *Pantoea citrea* 2-KDGDH enzyme.
Figure 5 depicts the amino acid sequence of subunit B (SEQ ID NO. 4) encoded by SEQ ID NO. 3.
Figure 6 depicts a nucleic acid sequence (SEQ ID NO. 5) encoding an amino acid sequence of subunit C of a *Pantoea citrea* 2-KDGDH enzyme.

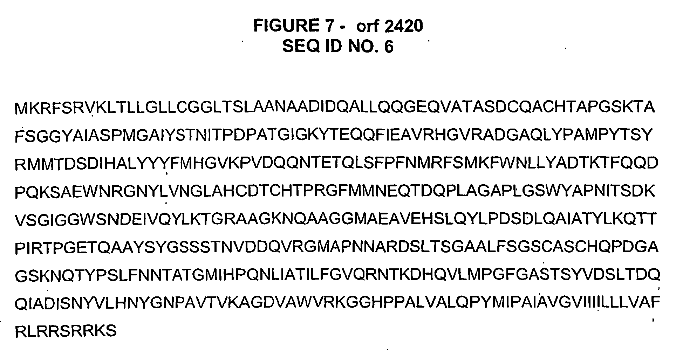

Figure 7 depicts the amino acid sequence of subunit C (SEQ ID NO. 6) encoded by SEQ ID NO. 5.

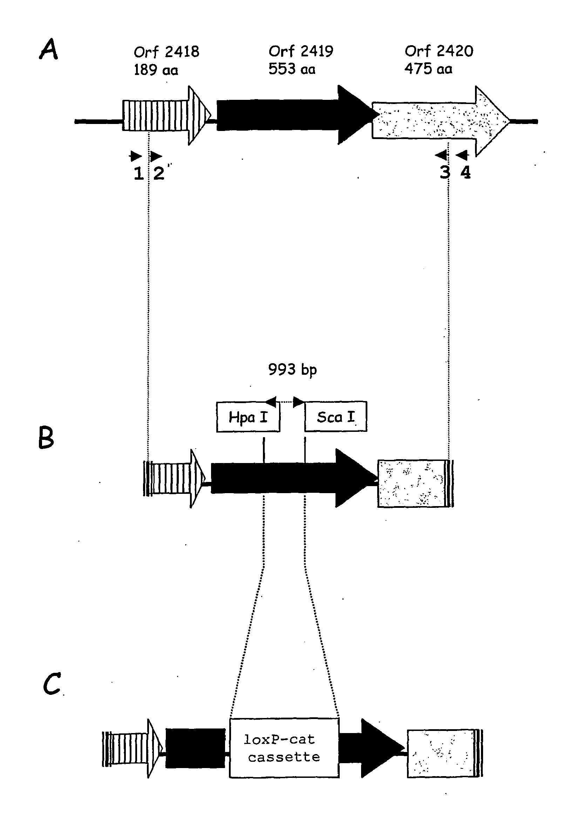

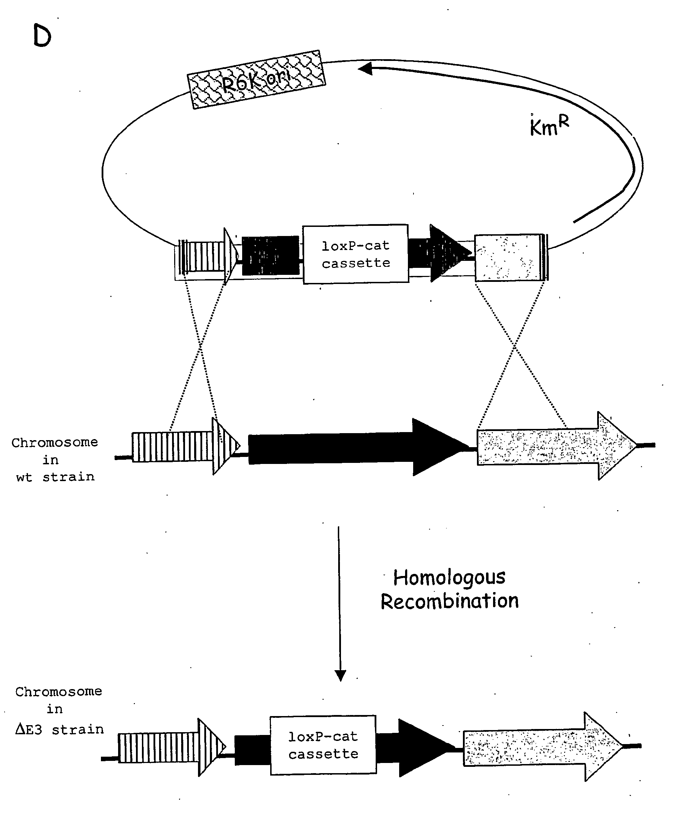

Figure 8 is a general schematic diagram illustrating the process used to inactivate a 2-KDGDH operon in *Pantoea citrea.* A represents the chromosomal 2-KDGDH operon which is comprised of three open reading frames (orfs). Orf 2418 represents subunit A, orf 2419 represents subunit B and orf 2420 represents subunit C. The arrows indicate the direction of primers wherein 1 represents primer KDGF2, 2 represents primer KDGF1, 3 represents primer KDGR1 and 4 represents primer KDGR2. B represents the PCR product resulting from the use of primers 2 and 3, including the restriction sites Hpal and Scal. The PCR product from B is used with a IoxP-cat cassette (C) and transferred into the non-replicating R6K vector which has a kanamycin resistant gene (D). The vector is transformed into a *Pantoea* host and homologous recombination is allowed to occur between the homologous regions of the vector and the homologous regions of the encoding region of the 2-KDGDH operon in the host chromosome. Reference is also made to example 1.
Figure 9 illustrates the formation of 2-KDG and 2,5-DKG by an altered bacterial strain encompassed by the invention (WKDG4) and an unaltered (isogenic wild-type) strain (139-2a/Ps-) as further discussed in example 2, wherein -■- represents 2-KDG accumulation in.WKDG4; -□- represents 2-KDG accumulation in 139-2a/Ps-; -•- represents 2,5-DKG accumulation in WKDG4 and -○- represents 2,5-DKG accumulation in 139-2a/Ps-.

### 4. DETAILED DESCRIPTION OF THE PREFERRED EMBODIMENTS

The practice of the present invention will employ, unless otherwise indicated, conventional techniques of molecular biology (including recombinant techniques), microbiology, cell biology, and biochemistry, which are within the skill of one in the art. Such techniques are explained fully in the literature, such as, MOLECULAR CLONING: A LABORATORY MANUAL, second edition (Sambrook et al., 1989) Cold Spring Harbor Laboratory Press; CURRENT PROTOCOLS IN MOLECULAR BIOLOGY (F.M. Ausubel et al., eds., 1987 and annual updates); OLIGONUCLEOTIDE SYNTHESIS (M.J. Gait, ed., 1984); PCR: THE POLYMERASE CHAIN REACTION, (Mullis et al., eds., 1994); MANUAL OF INDUSTRIAL MICROBIOLOGY AND BIOTECHNOLOGY, Second Edition (A.L. Demain, et al., eds. 1999); MANUAL OF METHODS FOR GENERAL BACTERIOLOGY (Phillipp Gerhardt, R. G. E. Murray, Ralph N. Costilow, Eugene W. Nester, Willis A. Wood, Noel R. Krieg and G. Briggs Phillips, eds), pp. 210-213 American Society for Microbiology, Washington, DC, and BIOTECHNOLOGY: A TEXTBOOK OF INDUSTRIAL MICROBIOLOGY, (Thomas D. Brock) Second Edition (1989) Sinauer Associates, Inc., Sunderland, Mass.

### A. Definitions.

Unless defined otherwise herein, all technical terms and scientific terms used herein have the same meaning as commonly understood by one of ordinary skill in the art to which this invention pertains. Singleton, et al., DICTIONARY OF MICROBIOLOGY AND MOLECULAR BIOLOGY, 2D ED., John Wiley and Sons, New York (1994) and Hale and Marham, THE HARPER DICTIONARY OF BIOLOGY, Harper Perennial, New York (1991) provide one of skill with general dictionaries of many of the terms used in this invention.

As used herein "2-keto-D-gluconate dehydrogenase (2-KDGDH)" and "an enzyme having 2-KDGDH activity" refer to a membrane-bound protein, which is capable of catalyzing the conversion of 2-KDG to 2,5-DKG in an oxidative pathway beginning with glucose or gluconate in a bacterial cell. The term 2-KDGDH has a functional definition and refers to any enzyme, which is membrane-bound and catalyzes the conversion of 2-KDG to 2, 5-DKG.

A "dehydrogenase protein" or "a protein having dehydrogenase activity" means an enzyme that catalyzes an oxidoreduction reaction involving removal of hydrogen from one substrate and its transfer to another molecule, usually to a coenzyme, such as nicotinamide adeninedinucleotide (NAD), nicotine adeninedinucleotide phosphate (NADP) and flavin adenine dinucleotide (FAD).

A "cytochrome C protein" refers to an electron transfer protein having one or several heme c groups bound to the protein by one or more, commonly two, thioether bonds involving sulphydryl groups of cysteine residues. The fifth heme iron ligand is always provided by a histidine amino acid residue. (Pettigrew et al. (1987) CYTOCHROMES C. BIOLOGICAL ASPECTS, Springer Verlag, Berlin; Moore et al. (1990) CYTOCHROMES C: EVOLUTIONARY, STRUCTURAL AND PHYSIOCHEMICAL ASPECTS. SpringerVerlag, Berlin; and Ambler (1991) Biochim. Biophys. Acta. 1058:42-47).

As used herein, the term "carbon source" encompasses suitable carbon substrates ordinarily used by microorganisms, such as 6 carbon sugars, including but not limited to glucose (G), gulose, lactose, sorbose, fructose, idose, galactose and mannose all in either D or L form, or a combination of 6 carbon sugars, such as glucose and fructose, and/or 6 carbon sugar acids including but not limited to 2-keto-L-gulonic acid, idonic acid (IA), gluconic acid (GA), 6-phosphogluconate, 2-keto-D-gluconic acid (2KDG), 5-keto-D-gluconic acid, 2-ketogluconatephosphate, 2, 5-diketo-L-gulonic acid, 2,3-L-diketogulonic acid, dehydroascorbic acid, erythorbic acid (EA) and D-mannonic acid.

The terms "non-functional", "inactivated" and "inactivation" when referring to an operon, gene or a protein means that the known normal function or activity of the operon, gene or protein has been eliminated, disrupted or highly diminished. Inactivation which renders the operon, gene or protein non-functional includes such methods as deletions, mutations; substitutions, interruptions or insertions in the nucleic acid sequence.

A "deletion" of an operon or gene as used herein refers to deletion of the entire coding sequence of one or more genes, deletion of part of the coding sequence of one or more genes, deletion of the regulatory region, deletion of the translational signals or deletion of the coding sequence including flanking regions of one or more genes.

As used herein the term "gene" means a DNA segment that is involved in producing a polypeptide and includes regions preceding and following the coding regions as well as intervening sequences (introns) between individual coding segments (exons).

The terms "polynucleotide" and "nucleic acid", used interchangeably herein, refer to a polymeric form of nucleotides of any length, either ribonucleotides or deoxyribonucleotides. These terms include a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs, uracyl, other sugars and linking groups such as fluororibose and thioate, and nucleotide branches. The sequence of nucleotides may be interrupted by non-nucleotide components.

The term "operon" as used herein means two or more genes which are transcribed as a single transcriptional unit from a common promoter. In some preferred embodiments, the genes comprising the operon are contiguous genes.

The term "promoter" as used herein refers to a nucleic acid sequence that functions to direct transcription of a downstream gene or genes.

"Under transcriptional control" or "transcriptionally controlled" are terms well understood in the art that indicate that transcription of a polynucleotide sequence, usually a DNA sequence, depends on its being operably linked to an element which contributes to the initiation of, or promotes, transcription.

"Operably linked" refers to a juxtaposition wherein the elements are in an arrangement allowing them to function. "Under translational control" is a term well understood in the art that indicates a regulatory process that occurs after the messenger RNA has been formed.

The term "over expressed" means an increased number of copies of the same gene product in a host cell.

As used herein when describing proteins, and genes that encode them, the term for the gene is not capitalized and is italics, i.e. *glkA*. The term for the protein is generally not italicized and the first letter is capitalized, i.e. GlkA.

The terms "protein" and "polypeptide" are used interchangeability herein.

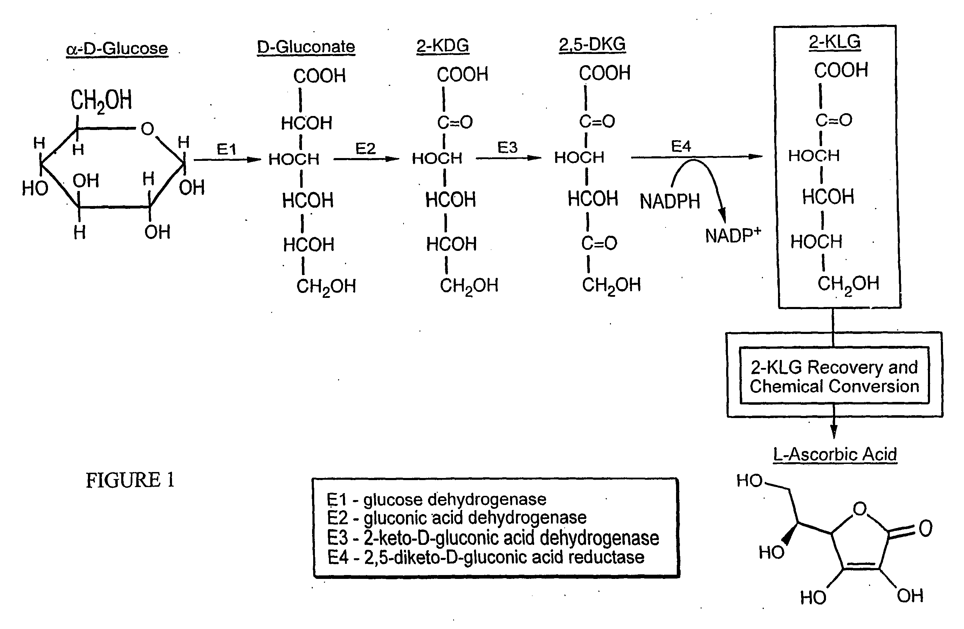

As used herein an "ascorbic acid (ASA) intermediate" means gluconate (GA); 2-keto-D-gluconate (2-KDG); 2,5-diketo-D-gluconate (2,5-DKG); 2-keto-L-gulonic acid (2-KLG); and L-idonic acid (IA). The chemical formulas for some of these compounds is shown in Figure 1.

As used herein "oxidative pathway" of a host cell means that a host cell comprises at least one enzyme that oxidizes a carbon source, such as D-glucose and/or its metabolites. An oxidative pathway in a host cell may comprise, one, two, three or more enzymes. An example of an oxidative pathway is the formation of gluconate from glucose through the activity of glucose dehydrogenase. Another example of an oxidative pathway is the formation of 2-KDG from glucose through the activity of glucose dehydrogenase and gluconate dehydrogenase. Another example of an oxidative pathway is the formation of 2,5-DKG from glucose through the activity of glucose dehydrogenase, gluconate dehydrogenase and 2-KDGDH.

As used herein "catabolic pathway" of a host cell means that a host cell comprises at least one enzyme that generates at least one metabolic intermediate. Very often, the formation of the metabolic intermediate is coupled to the generation of ATP, NADPH, or NADH for example by phosphorylating a carbon source such as D-glucose and/or its metabolites. An intracellular catabolic pathway in a host cell means the host cell comprises the activity of at least one enzyme in the host cell cytosol. In some embodiments, a catabolic pathway comprises the activity of two, three or more enzymes. Catabolic pathways include but are not limited to glycolysis, the pentose pathway and the TCA cycle pathway.

As used herein, the term "bacteria" refers to any group of microscopic organisms that are prokaryotic, i.e., that lack a membrane-bound nucleus and organelles. All bacteria are surrounded by a lipid membrane that regulates the flow of materials in and out of the cell. A rigid cell wall completely surrounds the bacterium and lies outside the membrane. There are many different types of bacteria, some of which include, but are not limited to *Bacillus, Streptomyces, Pseudomonas,* and strains within the families of Enterobacteriaceae.

As used herein, the family "Enterobacteriaceae " refers to bacterial strains having the general characteristics of being gram negative and being facultatively anaerobic. For the production of ASA intermediates, preferred Enterobacteriaceae strains are those that are able to produce 2,5-diketo-D-gluconic acid from D-glucose or carbon sources which can be converted to D-glucose by the strain. (Kageyama et al., International J. Sys. Bacteriol. 42:203 (1992)). Included in the family of *Enterobacteriaceae* are *Erwinia, Enterobacter, Gluconobacter, Klebsiella, Escherichia* and *Pantoea.* In the present invention, a preferred *Enterobacteriaceae* fermentation strain for the production of 2-KDG is a *Pantoea* species.

The genus *Pantoea* includes *P. agglomerans, P. dispersa, P. punctata, P. citrea, P. terrea, P. ananas* and *P. stewartii* and in particular, *Pantoea citrea. Pantoea citrea* can be obtained from ATCC (Manassas, VA) for example ATCC No. 39140. *Pantoea citrea* has sometimes been referred to as *Erwinia citreus* or *Acetobacter cerinus.* Thus, it is intended that the genus *Pantoea* include species that have been reclassified, including but not limited to *Erwinia citreus* or *Acetobacter cerinus.*

As used herein the family *"Bacillus"* refers to rod-shaped bacterial strains having the general characteristics of being gram positive and capable of producing resistant endospores in the presence of oxygen. Examples of *Bacillus* include *B. subtilis, B. licheniformis, B. lentus, B. circulans, B. lautus, B. amyloliquefaciens, B. stearothermophilus, B. alkalophilus, B. coagulans, B. thuringiensis* and *B. brevis.*

An "altered bacterial host" or "altered bacterial cell" according to the invention refers to a bacterial cell, which was capable of enzymatically converting 2-KDG to 2,5-DKG by the action of an endogenous 2-KDGDH and wherein the endogenous 2-KDGDH enzyme has been rendered non-functional. In one embodiment, an altered bacterial cell will have a higher level of accumulated 2-KDG compared to the level of accumulated (or transiently accumulated) 2-KDG in a corresponding unaltered bacterial host cell grown under essentially the same culture conditions.

An "unaltered bacterial cell" or "unaltered bacterial host" according to the invention is a bacterial cell wherein endogenous 2-KDGDH is not inactivated, and the 2-KDGDH enzyme remains functional resulting in the oxidation of 2-KDG to 2,5-DKG.

"Higher level of accumulated 2-KDG" refers to an amount of 2-KDG in an altered bacterial cell compared to the amount of 2-KDG in a corresponding unaltered bacterial host cell when cultured under essentially the same conditions. For example, a higher level of accumulated 2-KDG may be an increase of at least 1%, 2%, 5%, 10%, 15%, 20%, 30%, 40%, 50%, 60% or more over the amount of 2-KDG produced in the corresponding unaltered bacterial host. The increase in the level of accumulation may be expressed in numerous ways including as a weight % (gm product/gm substrate) or gm/L per unit of time. The higher level of accumulated 2-KDG results from the inactivation of one or more genes necessary to produce a functional 2-KDGDH.

As used herein "chromosomal integration" is a process whereby an introduced polynucleotide is incorporated into a host cell chromosome. The process preferably takes place by homologous recombination.

As used herein, "modifying" the level of protein or enzyme activity produced by a host cell refers to controlling the levels of protein or enzymatic activity produced during culturing, such that the levels are increased or decreased as desired.

As used herein, the term "modified" when referring to nucleic acid or a polynucleotide means that the nucleic acid has been altered in some way as compared to a wild type nucleic acid, such as by mutation in; deletion of part or all of the nucleic acid; or by being operably linked to a transcriptional control region. As used herein the term "mutation" when referring to a nucleic acid refers to any alteration in a nucleic acid such that the product of that nucleic acid is partially or totally inactivated. Examples of mutations include but are not limited to point mutations, frame shift mutations and deletions of part or all of a gene or genes encoding a 2-KDGDH.

"Desired product" as used herein refers to the desired compound to which a carbon substrate is bioconverted into. Exemplary desired products are gluconic acid, 2-KDG; and 5-keto-D-gluconate.

As used herein, the term "recombinant" when used in reference to a cell, nucleic acid or protein indicates the cell, nucleic acid or protein has been modified by the introduction of a heterologous nucleic acid or the alteration of a native nucleic acid or that the cell is derived from a cell so modified. Thus, for example, recombinant cells express gene that are not found within the native (non-recombinant) form of the cell or express native genes that are otherwise under expressed or not expressed at all. In some embodiment the altered bacterial host cells of the invention are recombinant cells.

As used herein, the term "endogenous" refers to a nucleic acid or protein encoded by a nucleic acid naturally occurring in the host.

The term "heterologous" as used herein refers to nucleic acid or amino acid sequences not naturally occurring in the host cell.

The terms "isolated" or "purified" as used herein refer to an enzyme, nucleic acid, protein, peptide or co-factor that is removed from at least one component with which it is naturally associated.

As used herein, the term "vector" refers to a polynucleotide construct designed to introduce nucleic acids into one or more cell types. Vectors include cloning vectors, expression vectors, shuttle vectors, plasmids, cassettes and the like.

A polynucleotide or polypeptide having a certain percentage (for example, 80%, 85%, 90%, 95%, 96%, 97% or 99%) of "sequence identity" to another sequence means that, when aligned, that percentage of bases or amino acids are the same in comparing the two sequences. This alignment and the percent homology or sequence identity can be determined using software programs known in the art, for example those described in CURRENT PROTOCOLS IN MOLECULAR BIOLOGY (F.M. Ausubel et al., eds., 1987) Supplement 30, section 7.7.18. A preferred alignment program is ALIGN Plus (Scientific and Educational Software, Pennsylvania), preferably using default parameters, which are as follows: mismatch = 2; open gap = 0; extend gap = 2. Another sequence software program that could be used is the TFastA Data Searching Program available in the Sequence Analysis Software Package Version 6.0 (Genetic Computer Group, University of Wisconsin, Madison, WI).

It is well understood in the art that the acidic derivatives of saccharides, may exist in a variety of ionization states depending upon their surrounding media, if in solution, or out of solution from which they are prepared if in solid form. The use of a term, such as, for example, gluconic acid, to designate such molecules is intended to include all ionization states of the organic molecule referred to. Thus, for example, "gluconic acid" and "gluconate" refer to the same organic moiety, and are not intended to specify particular ionization states or chemical forms.

The term "culturing" as used herein refers to fermentative bioconversion of a carbon substrate to a desired product within a reactor vessel. Bioconversion means contacting a microorganism with a carbon substrate to convert the carbon substrate to the desired product.

As used herein, the term "comprising" and its cognates are used in their inclusive sense; that is, equivalent to the term "including" and its corresponding cognates.

"A," "an" and "the" include plural references unless the context clearly dictates otherwise.

The term "transporter" as used herein refers to a protein that catalyzes the transport of a molecule across the internal cell membrane. A glucose transporter catalyzes the transport of glucose into the cytoplasm. A gluconate transporter catalyzes the transport of gluconate and may also catalyze the transport of other sugar acid molecules or sugar-keto acids across a cell membrane.

"Cytoplasm" or "cytoplasmic" refers to being within the inner cell membrane. Extracellular or outside the inner cell membrane refers to cell locations on the opposite side of a membrane from the cytoplasm, including but not limited to the periplasm. Internal or inner membrane (and sometimes referred to as the periplasmic membrane) refers to the barrier that separates the cytoplasm from the periplasm. Intracellular refers to the portion of the cell on the side of the membrane that is closest to the cytosol. Intracellular includes cystolic.

As used herein, the phrase "glucokinase" (E.C. -2.7.1.2) means an enzyme which phosphorylates D-glucose or L-glucose at its 6th carbon, for example GlkA.

As used herein, the phrase "gluconokinase" (E.C. - 2.7.1.12) means an enzyme which phosphorylates D-gluconate or L-gluconate at its 6^{th} carbon, for example GntK.

### B. Preferred Embodiments.

### Polynucleotides and proteins:

The present application concerns isolated polynucleotides encoding a 2-KDGDH enzyme. Many bacterial species have been found to contain a 2-KDGDH enzyme. The Nomenclature Committee of the International Union of Biochemistry and Molecular Biology (NC-IUBMB) has assigned number EC 1.1.99.4 to 2-KDGDH. More specifically, 2-KDGDH belongs to the medium-chain dehydrogenase/reductase (MDR) class of enzymes. This is a large enzyme superfamily with approximately one thousand (1000) members. 2-KDGDH has been classified in the MDR branch which exhibits polyol dehydrogenase (PDH) activities. (Nordling et al., (2002) Eur. J. Biochem. 269:4267-4276).

The 2-KDGDH enzyme is preferably a membrane-bound enzyme isolated from a *Pantoea, Acetobacter, Erwinia or Gluconobacter* species, and particularly from *Pantoea citrea* or *Pantoea agglomerans.* These species have been shown to produce 2-5-DKG from the conversion of 2-KDG and gluconate.

In one embodiment, the 2-KDGDH enzyme is a multimeric complex encoded by an operon comprising three genes. Preferably the three genes are contiguous and are organized as a single transcriptional unit. The first gene of the operon encodes a protein designated subunit A. The second gene of the operon encodes a protein designated subunit B, wherein subunit B is a dehydrogenase protein. In one preferred embodiment, the subunit B dehydrogenase is a flavoprotein. Flavoproteins are enzymes that contain a derivative of riboflavin as a prosthetic group. The third gene of the operon encodes a protein designated subunit C, wherein subunit C is a cytochrome c protein.

In one embodiment, an isolated polynucleotide encodes a subunit A protein having the amino acid sequence of SEQ ID NO. 2 or an amino acid having at least 95%, 96%, 97%, 98% and 99% sequence identity with SEQ ID NO. 2 In another embodiment, an isolated polynucleotide encodes a subunit B dehydrogenase protein having the amino acid sequence of SEQ ID NO. 4 or an amino acid having at least 95%, 96%, 97%, 98% and 99% sequence identity with SEQ ID NO. 4. In a further embodiment, an isolated polynucleotide encodes a subunit C cytochrome C protein having the amino acid sequence of SEQ ID NO. 6 or an amino acid having at least 95%, 96%, 97%, 98% and 99% sequence identity with SEQ ID NO. 2. One of skill in the art is well aware of the degeneracy of the genetic code and that an amino acid may be coded for by more than one codon. These variations are include as part of the invention herein.

In a further embodiment, the isolated polynucleotide which encodes subunit A of the 2-KDGDH complex comprises the nucleic acid sequence of SEQ ID NO. 1; the isolated polynucleotide which encodes the dehydrogenase protein designated as subunit B comprises the nucleic acid sequence of SEQ ID NO. 3 and the isolated polynucleotide which encodes the cytochrome C protein designated as subunit C comprises the nucleic acid sequence of SEQ ID NO. 5.

The invention also provides a method of using the polynucleotide sequences set forth in SEQ ID NOs. 1, 3 or 5 as probes for detecting 2-KDGDH enzymes in other microbial organisms. In one embodiment at least 10, 15, 20, 25, 30, 40, 50 our more contiguous sequences from anyone of SEQ ID NOs. 1, 3 or 5 may be used as a probe to detect a polynucleotides encoding subunit A, subunit B or subunit C of a 2-KDGDH enzyme complex. In another embodiment, at least 20 contiguous sequences from anyone of SEQ ID NOs. 1, 3 or 5 may be used as a probe. Further at least 10, 15, 20, 25, 30, 40, 50 or more contiguous sequences from SEQ ID NO. 3 may be used as a probe. Additionally oligonucleotide probes useful in the present invention may comprise a nucleic acid sequence encoding a polypeptide having at least 5, 10, 15, 20, 25, 30 or more contiguous amino acid residues of SEQ ID NOs. 2, 4 or 6.

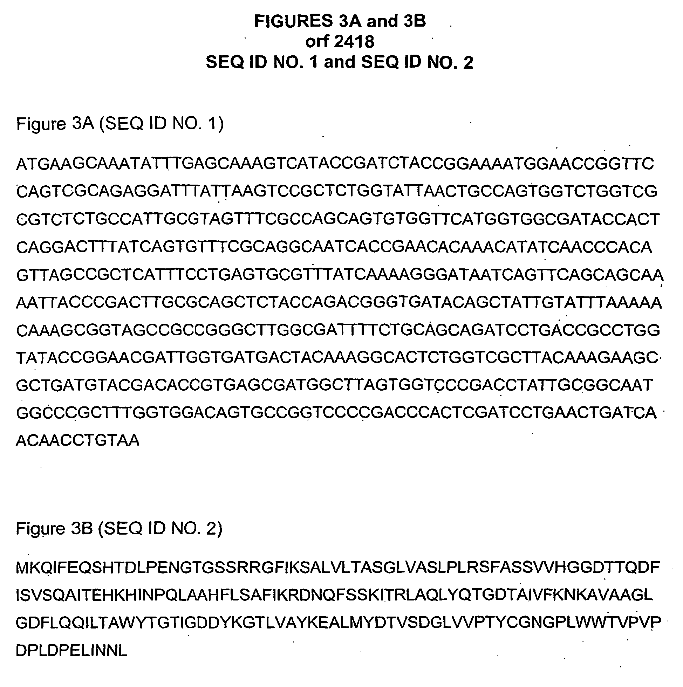

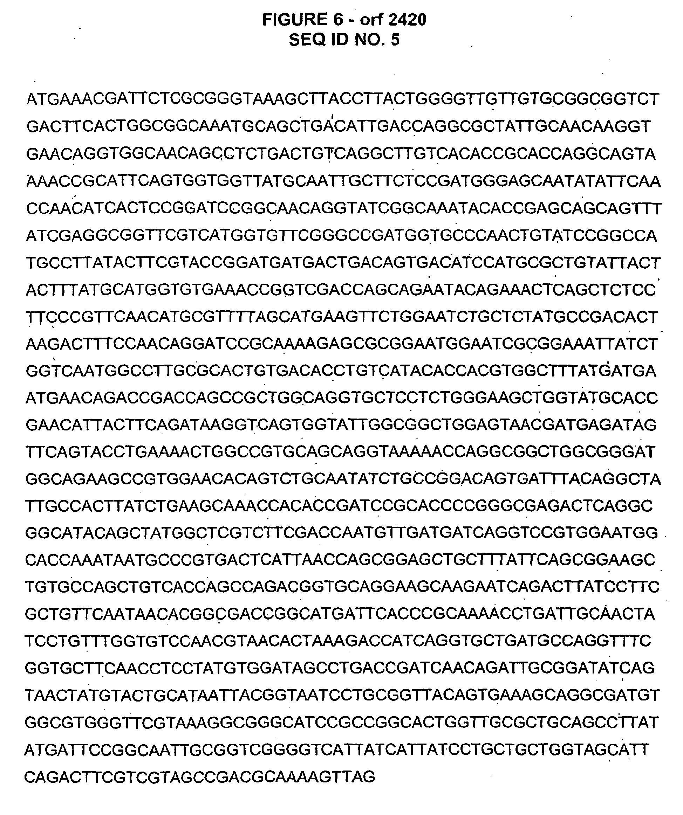

Methods for identifying 2-KDGDH enzymes or 2-KDGDH enzyme subunits found in other bacterial microorganisms and specifically in Pantoea species are known in the art and would include hybridization studies. Thus, for example, nucleic acid sequences which hybridize under high stringency conditions to a sequence disclosed in Figures 3A, 4 or 6 or a complement thereof, may also encode proteins that function as subunit A, subunit B or subunit C of a 2-KDGDH enzyme. Hybridization includes the process by which a strand of nucleic acid joins with a complementary strand through base pairing. High stringency conditions are known in the art and see for example, Maniatis, et al., MOLECULAR CLONING: A LABORATORY MANUAL, 2d Edition (1989) and SHORT PROTOCOLS IN MOLECULAR BIOLOGY, ed Ausubel et al. Stringent conditions are sequence dependent and will be different in different circumstances. Longer sequences hybridize specifically at higher temperatures. An extensive guide to the hybridization of nucleic acids is found in Tijssen, TECHNIQUES IN BIOCHEMISTRY AND MOLECULAR BIOLOGY - HYBRIDIZATION WITH NUCLEIC ACID PROBES, Overview of Principles of Hybridization and the Strategy of Nucleic Acid Assays (1993). Generally stringent conditions are selected to be about 5 to 10 degrees lower than the thermal melting point Tm for the specific sequence at a defined ionic strength and pH. The Tm is the temperature (under defined ionic strength, pH and nucleic acid concentration) at which 50% of the probes complementary to the target, hybridize to the target sequence at equilibrium. Stringent conditions will be those in which the salt concentration is less than about 1.0 M sodium ion, typically about 0.01 to 1.0M sodium ion concentration (or other salts) at pH 7.0 to 8.3 and the temperature is at least 30°C for short probes (e.g. for 10 to 50 nucleotides) and at least about 60°C for long probes (e.g. greater than 50 nucleotides). Stringent conditions may also be achieved with the addition of destabilizing agents such as formamide. In another embodiment, less stringent hybridization conditions are used. For example moderate or low stringent conditions may be used.

Polymerase chain reaction (PCR) may also be used to screen for homologous sequences and reference is made to Chen et al., (1995) Biotechniques 18(4):609-612. Other methods include protein bioassay or immunoassay techniques which include membrane-based, solution-based, or chip-based technologies for the detection and/or quantification of the nucleic acid or protein.

Isolated cytochrome c protein has the amino acid sequence set forth in SEQ ID NO. 6 and an isolated dehydrogenase having the amino acid sequence set forth in SEQ ID NO. 4. Subunit A having the nucleic acid sequence set forth in SEQ ID NO.1, which encodes the amino acid sequence set forth in SEQ ID NO. 2, has 89.6% identity and 95.8% identity respectively with a polynucleotide encoding a keto-D-gluconate dehydrogenase subunit precursor disclosed in Pujol and Kado (2000) J. Bacteriol. 182:2230 - 2237. Subunit B having the nucleic acid sequence set forth in SEQ ID NO. 3, which encodes the amino acid sequence set forth in SEQ ID NO. 4, has 89.9% identity and 99.8% identity respectively with a polynucleotide encoding a keto-D-gluconate dehydrogenase subunit precursor disclosed in Pujol and Kado (2000), *supra.* Subunit C having the nucleic acid sequence set forth in SEQ ID NO. 5, which encodes the amino acid sequence as set forth in SEQ ID NO. 6 has 85.5% identity and 94.3% identity with a polynucleotide encoding the keto-D gluconate dehydrogenase subunit precursor disclosed in Pujol and Kado (2000), *supra.* The nucleotide sequence of the putative 2-ketogluconate dehydrogenase operon disclosed in Pujol and Kado has been deposited with GenBank with accession no. AF131202.

Dehydrogenase assays are well known and may be adopted from the methods described in Bouvet et al. (1989) Int. J. Syst. Bacteriol. 39:61 - 67 using cells grown on MGY supplemented with 2KDG. Reference is also made to Shinagawa and Ameyama (1982) Meth. Enzymol. 89:194 - 198.

Further qualitative assays for detection of gluconate, 2-KDG and 2,5-DKG are well known in the art, and for example may be obtained by use of HPLC.

### Methods of inactivating 2-KDGDH enzymes.

In general, methods of rendering chromosomal genes non-functional are well known in the art and a number of these techniques may be used to inactivate an enzyme having 2-KDGDH activity. Some of these methods include genetic engineering techniques and other methods include techniques such as screening and mutagenesis.

A 2-KDGDH enzyme may be rendered non-functional by inactivation of any one of the three subunits which comprise the 2-KDGDH enzyme complex. These subunits have been designated subunit A, subunit B having dehydrogenase activity and subunit C (a cytochrome c protein).

In one preferred embodiment, subunit B is rendered non-functional, and it is the inactivation of this subunit which results in inactivation of a 2-KDGDH enzyme. In another embodiment, subunit C is rendered non-functional, and it is the inactivation of this subunit which results in inactivation of a 2-KDGDH enzyme. In another embodiment, subunit A is rendered non-functional, and it is the inactivation of this subunit which results in inactivation of a 2-KDGDH enzyme.

In further embodiments two subunits are rendered non-functional, for example both subunit B and subunit C and this results in inactivation of the 2-KDGDH enzyme. In a further embodiment, the expression product of an inactivated gene, such as a deleted gene, may be a truncated protein as long as the protein has a change in its biological activity. The change in biological activity could be an altered activity, but preferably is loss of biological activity.

One preferred method of inactivation is a deletion of at least one gene encoding one of the subunits of a 2-KDGDH enzyme. In one embodiment, the gene to be deleted encodes subunit B, a dehydrogenase enzyme. In another embodiment, the gene to be deleted encodes subunit C, a cytochrome C protein. In another embodiment, the gene to be deleted encodes subunit A. In a further embodiment, two genes may be deleted for example the genes encoding subunit B and subunit C. Preferably, the gene encoding subunit B is inactivated by deletion: In all instances, the deletion may be partial as long as the sequences left in the chromosome are too short for biological activity of the gene in issue.

One method of deleting a 2-KDGDH enzyme subunit according to the invention includes constructing a vector which includes homologous flanking regions of the subunit coding sequence of interest. The flanking regions may include from about 1 bp to about 500 bp at the 5' and 3' ends. The flanking region of the vector may be larger than 500 bp and in certain embodiments may include other genes encompassing the 2-KDGDH operon resulting in the inactivation or deletion of these genes as well. The vector construct may be introduced into a host cell by, for example, transformation and then integrated into the host cell chromosome. The end result is that the introduced DNA causes the deletion of one or more endogenous genes which encode the 2-KDGDH enzyme thus resulting in a non-functional 2-KDGDH enzyme.

For example, as shown in Figure 8, an inactivation cassette is constructed by first cloning a DNA fragment containing a 2-KDGDH subunit B (2-KDGDH-B) gene into a vector. To inactivate the 2-KDGDH-B gene, an antibiotic resistance gene (i.e. a chloramphenicol, Cm^{R} gene) is cloned into a unique restriction site found in the 2-KDGDH-B gene. The insertion of the antibiotic marker into the 2-KDGDH-B gene interrupts its normal coding sequence. The inactivation cassette is transferred to the host cell chromosome by homologous recombination using a non-replication R6K vector like pGP704 (Miller et al. (1988) J. Bacteriol. 170:2575-2583). The transfer of the cassette into the host cell chromosome is selected by the inclusion of Cm^{R} by the host cell. Once the inactivation of the 2-KDGDH-B gene has been corroborated, the Cm^{R} is removed from the 2-KDGDH-B coding region leaving an interrupting spacer (which in this example includes a copy of a IoxP site) in the coding region, inactivating the coding region. (Palmeros et al., (2000) Gene 247:255 - 264).

In another embodiment, inactivation is by insertion. Insertional inactivation includes interruption of the chromosomal coding region. For example when the gene encoding subunit B is the gene to be inactivated, a DNA construct will comprise a nucleic acid sequence encoding subunit B wherein the coding sequence is interrupted by a selective marker. The selective marker will be flanked on each side by sections of the subunit B coding sequence. The DNA construct aligns with essentially identical sequences of the subunit B gene in the host chromosome and in a double crossover event the subunit B gene is inactivated by the insertion of the selective marker. A selectable marker refers to a gene capable of expression in the host microorganism which allows for ease of selection of those hosts containing the vector. Examples of such selectable markers include but are not limited to antibiotic resistant genes such as, erythromycin, kanamycin, chloramphenicol and tetracycline. The process as generally delineated above could be performed with the sequences encoding subunit A, subunit B or subunit C.

Plasmids which can be used as vectors in bacterial organisms are well known and reference is made to Maniatis, et al., MOLECULAR CLONING: A LABORATORY MANUAL, 2d Edition (1989) AND MOLECULAR CLONING: A LABORATORY MANUAL, second edition (Sambrook et al., 1989) and Bron, S, Chapter 3, Plasmids, in MOLECULAR BIOLOGY METHODS FOR BACILLUS, Ed. Harwood and Cutting, (1990) John Wiley & Sons Ltd.

A preferred plasmid for the recombinant introduction of polynucleotides encoding non-naturally occurring proteins or enzymes into a strain of Enterobacteriaceae is RSF1010, a mobilizable, but not self transmissible plasmid which has the capability to replicate in a broad range of bacterial hosts, including Gram - and Gram+ bacteria. (Frey et al., 1989, The Molecular Biology of IncQ Plasmids. In: Thomas,(Ed.), PROMISCUOUS PLASMIDS OF GRAM NEGATIVE BACTERIA. Academic Press, London, pp. 79-94). Frey et al. report on three regions found to affect the mobilization properties of RSF1010 (Frey et al. (1992) Gene 113:101-106).

In another embodiment, inactivation is by insertion in a single crossover event with a plasmid as the vector. For example, a chromosomal gene encoding a 2-KDGDH subunit such as subunit B having a nucleic acid sequence as disclosed in SEQ ID NO. 3 is aligned with a plasmid comprising the sequence of SEQ ID NO. 3 or part of the gene coding sequence and a selective marker. The selective marker may be located within the gene coding sequence or on a part of the plasmid separate from the gene. The vector may be integrated into the bacterial chromosome, and the gene is inactivated by the insertion of the vector in the coding sequence.

Inactivation may also occur by a mutation of the gene. Methods of mutating genes are well known in the art and include but are not limited to chemical mutagenesis, site-directed mutation, generation of random mutations, and gapped-duplex approaches. ( USP 4,760,025; Moring et al., Biotech. 2:646 (1984); and Kramer et al., Nucleic Acids Res. 12:9441 (1984)). Another well known approach includes the use of random mutagenesis by transposons (Miller J.H. (1992) A SHORT COURSE IN BACTERIAL GENETICS, Cold Spring Harbor Laboratory Press, New York pp 372 - 380). After mutagenesis has occurred, mutants having the desired properties may be selected by a variety of methods, for example selective isolation on a selective medium.

In some embodiments, a mutagenic vector is constructed which contains a multiple cloning site cassette which preferably comprises at least one restriction endonuclease site unique to the vector, to facilitate ease of nucleic acid manipulation. In a preferred embodiment, the vector also comprises one or more selectable markers.

In an altered bacterial strain, the inactivation of the 2-KDGDH enzyme will preferably be a stable and non-reverting inactivation.

### Host cells.

The method of the present invention employ *Pantoea* cells.

Particularly preferred *Pantoea* cells are *P. citrea* and *P. aggolmerans.* Sources of such microorganisms include public repositories, such as ATCC and commercial sources. For example ATCC accession numbers 21998, 13182, and 39140. Further reference is made to USP 5,032,514 and Truesdell et al., (1991) J. Bacteriol. 173:6651 - 6656. *Bacillus* sp. may also serve as host cells. An altered bacterial host cell comprising a non-functional 2-KDGDH enzyme may be a recombinant host cell.

In one embodiment, an altered bacterial cell which includes an inactivated 2-KDGDH enzyme wherein the 2-KDGDH enzyme prior to in activation was capable of catalyzing the conversion of 2-KDG to 2, 5-DKG comprises an operon which incudes three genes encoding three protein subunits, designated subunit A, subunit B and subunit C. A modification such as a deletion of any one subunit or a part of any one subunit may render the endogenous 2-KDGDH non-functional.

In preferred embodiments, it is subunit C and/or subunit B that is modified to obtain the altered bacterial cells of the invention. Preferably subunit B is modified by either deletion of a part of the coding region or by insertional inactivation.

In one embodiment subunit B which is a dehydrogenase protein is encoded by a polynucleotide having the sequence set forth in SEQ ID NO. 3 or a polynucleotide having a nucleic acid sequence at least 70%, 75%, 80%, 85%, 90%, 95%, 96%, 97% or 99% identical to the sequence of SEQ ID NO. 3. In a further embodiment, subunit C which is a cytochrome c protein is encoded by a polynucleotide having the sequence set forth in SEQ ID NO. 5 or a polynucleotide having a nucleic acid sequence at least 70%, 75%, 80%, 85%, 90%, 95%, 96%, 97% or 99% identical to the sequence of SEQ ID NO. 5.

As a result of inactivation of the 2-KDGDH enzyme, regardless of whether the inactivation is due to inactivation or deletion of for example one, two or three subunits of the 2-KDGDH enzyme complex, the conversion of 2-KDG to 2,5 DKG will be diminished and 2-KDG will accumulate in the cell environment. According to this aspect of then invention an altered bacterial cell will have an increased or higher level of accumulated 2-KDG compared to a corresponding unaltered bacterial host cell, wherein 2-KDG is transiently accumulated.

### Additional gene modifications.

As stated above, bacterial host cells may be recombinant host cells. Modifications to host cells may have been realized prior to, simultaneously with, or after inactivation of a 2-KDGDH. The modifications may be made to chromosomal genes and the modifications may include inactivations, such as deletions or interruptions of endogenous chromosomal genes, modifications resulting in increased expression of endogenous chromosomal genes, and inclusion of heterologous genes.

More specific examples of additional modifications which may be incorporated into a bacterial host cell encompassed by the invention include but are not limited to: i) modifications to genes encoding gluconate transporters; ii) modifications to genes encoding glucose transporters (Peekhaus et al., 1997, Fems Micro. Lett. 147:233-238); iii) modifications to genes encoding KDG transporters; iv) gene modifications resulting in gene overexpression, for example overexpression of glucose dehydrogenase genes, overexpression of gluconate dehydrogenase genes, or genes involved in the biogenesis of cytochrome c such as *ccm* genes (Kranz et al., (1996) Mol. Microbiol. 29:3283 - 396); v) modifications to polynucleotides that uncouple the catabolic pathway from the oxidative pathway such as by inactivating an enzyme that phosphorylates D-glucose or D-gluconate at its 6th carbon and more specifically inactivating a hexokinase gene, a glucokinase gene or a gluconokinase gene i.e. *gntK* and/or *glkA* and reference is made to WO 02/081440; and vi) modifications to gene encoding enzymes that use 2-KDG as a substrate, for example 2-keto-reductases or 2-keto-kinases.

In certain preferred embodiments, an altered bacterial strain will include an inactivated *gnt*K and/or an inactivated *glk*A gene. In another preferred embodiment, an altered bacterial strain according to the invention will include an inactivated gluconate transporter.

In certain embodiments, the altered bacterial strain comprising an inactivated 2-KDGDH may additionally include other inactivated genes, for example two inactivated genes, three inactivated genes, four inactivated genes, five inactivated genes, six inactivated genes or more. The inactivated genes may be contiguous to one another or may be located in separate regions of the bacterial chromosome. An inactivated chromosomal gene may have a necessary function under certain conditions, but the gene is not necessary for bacterial strain viability under laboratory conditions. Preferred laboratory conditions include but are not limited to conditions such as growth in a fermentator, in a shake flask, in plate media or the like.

In one embodiment an altered bacterial cell comprising an inactivated 2-KDGDH may further comprise an inactivated glucokinase gene. In another embodiment, the host cell may be engineered to include genes encoding enzymes known to effect the conversion of glucose or other ordinary metabolites to 2-KDG or 2-KLG from the organisms known to contain them. Examples of the enzymes effecting the conversion of an ordinary metabolite to 2-KDG or 2-KLG are D-glucose dehydrogenase (Adachi, O. et al., (1980) Agric. Biol. Chem., 44:301-308; Ameyama, M. et al., (1981) Agric. Biol. Chem. 45:851-861; Smith et al. (1989) Biochem. J. 261:973; and Neijssel et al., (1989) Antonie Van Leauvenhoek 56(1):51-61); and D-gluconate dehydrogenase (Mclntire, W. et al., (1985) Biochem. J., 231:651-654; Shinagawa, E. et al., (1976) Agric. Biol. Chem. 40:475-483; Shinagawa, E. et al., (1978) Agric. Biol. Chem. 42:1055-1057; and Matsushita et al. (1979), J. Biochem. 85:1173); 5-keto-D-gluconate dehydrogenase (Shinagawa, E. et al., (1981) Agric. Biol. Chem., 45:1079-1085 and Stroshane (1977) Biotechnol. BioEng. 19(4) 459); and 2,5-diketo-D-gluconic acid reductase (USP Nos: 5,795,761; 5,376,544; 5,583,025; 4,757,012; 4,758,514; 5,008,193; 5,004,690; and 5,032,514). In another embodiment, the host cell may be engineered to include genes encoding enzymes that convert 2-KDG into other commercially useful products.

In some embodiments, the recombinant strain will optionally comprise either singly or in combination an inactivated gluconate transporter gene; an inactivated glucokinase gene; an inactivated gluconokinase gene; an inactivated glycerol kinase gene; and an inactivated glucose transport system. If gluconate is not transported across a cellular membrane there may be more gluconate available for conversion to 2-KDG in the oxidative pathway.

### Recovery, identification and purification of ASA intermediates.

Methods for detection of ASA intermediates, ASA and ASA stereoisomers such as erythorbic acid (D-araboascorbic acid), L-araboascorbic acid, and D-xyloascorbic acid include the use of redox-titration with 2,6 dichloroindophenol (Burton et al. (1979) J. Assoc. Pub. Analysts 17:105) or other suitable reagents; high-performance liquid chromatography (HPLC) using anion exchange; and electro-redox procedures (Pachia, (1976) Anal. Chem. 48:364). The skilled artisan will be well aware of controls to be applied in utilizing these detection methods. Alternatively, the intermediates can also be formulated directly from the fermentation broth or bioreactor and granulated or put in a liquid formulation. 2-KDG produced and accumulated according to the invention may be further converted to erythorbic by means known to those of skill in the art, see for example, Reichstein and Grussner, Helv. Chim. Acta., 17, 311-328 (1934).

### Gene transfer.

Gene transfer techniques for bacterial cells are well known and these techniques include transformation, transduction, conjugation and protoplast fusion. Gene transfer is the process of transferring a gene or polynucleotide to a cell or cells wherein exogenously added DNA is taken up by a bacterium. General transformation procedures are taught in CURRENT PROTOCOLS IN MOLECULAR BIOLOGY (vol. 1, edited by Ausubel et al., John Wiley & Sons, Inc. 1987, Chapter 9) and include calcium phosphate methods, transformation using DEAE-Dextran and electroporation. A variety of transformation procedures are known by those of skill in the art for introducing nucleic acids in a given host cell. (Reference is also made to USP 5,032,514; Potter H. (1988) Anal. Biochem 174:361-373; Sambrook, J. et al., MOLECULAR CLONING: A LABORATORY MANUAL, Cold Spring Harbor Laboratory Press (1989); and Ferrari et al., Genetics, pgs 57 - 72 in BACULUS, Harwood et al., Eds. Plenum Publishing Corp).

Transformation of a host cell can be detected by the presence/absence of marker gene expression which can suggest whether the nucleic acid of interest is present. However, its expression should be confirmed. For example, if the nucleic acid encoding a subunit B dehydrogenase protein is inserted within a marker gene sequence, recombinant cells containing the insert can be identified by the absence of marker gene function. Alternatively, a marker gene can be placed in tandem with nucleic acid encoding the dehydrogenase under the control of a single promoter. Expression of the marker gene in response to induction or selection usually indicates expression of the protein or enzyme as well. Once a bacterial microorganism capable of carrying out the conversion as described above has been created, the process of the invention may be carried out in a variety of ways depending on the nature of the construction of the expression vectors for the recombinant proteins and upon the growth characteristics of the host.

### Cell cultures and fermentations.

Methods suitable for the maintenance and growth of bacterial cells are well known and reference is made to the MANUAL OF METHODS OF GENERAL BACTERIOLOGY, Eds. P. Gerhardt et al., American Society for Microbiology, Washington DC (1981) and T.D. Brock in BIOTECHNOLOGY: A TEXTBOOK OF INDUSTRIAL MICROBIOLOGY, 2nd ed. (1989) Sinauer Associates, Sunderland, MA.

Cell Precultures -Typically cell cultures are grown at 25 to 32°C, and preferably about 28 or 29°C in appropriate media. Exemplary growth media useful in the present invention are common commercially prepared media such as but not limited to Luria Bertani (LB) broth, Sabouraud Dextrose (SD) broth or Yeast medium (YM) broth. These may be obtained from for example, GIBCO/BRL (Gaithersburg, MD). Other defined or synthetic growth media may be used and the appropriate medium for growth of the particular bacterial microorganism will be known by one skilled in the art of microbiology or fermentation science. Suitable pH ranges preferred for the fermentation are between pH 5 to pH 8. Preferred ranges for seed flasks are pH 7 to pH 7.5 and preferred ranges for the reactor vessels are pH 5 to pH 6. It will be appreciated by one of skill in the art of fermentation microbiology that a number of factors affecting the fermentation processes may have to be optimized and controlled in order to maximize the ascorbic acid intermediate production. Many of these factors such as pH, carbon source concentration, and dissolved oxygen levels may affect enzymatic processes depending on the cell types used for ascorbic acid intermediate production.

The production of ASA intermediates can proceed in a fermentative environment, that is, in an *in vivo* environment, or a non-fermentative environment, that is, in an *in vitro* environment; or combined *in vivo*/*in vitro* environments. The fermentation or bioreactor may be performed in a batch process or in a continuous process.

### In vivo biocatalytic environment:

Biocatalysis begins with culturing an altered bacterial host cell according to the invention in an environment with a suitable carbon source ordinarily used by *Enterobacteriaceae* or other bacterial strains. Suitable carbon sources include 6 carbon sugars, for example, glucose, or a 6 carbon sugar acid, or combinations of 6 carbon sugars and/or 6 carbon sugar acids. Other carbon sources include, but are not limited to galactose, lactose, fructose, or the enzymatic derivatives of such.

In addition, fermentation media must contain suitable carbon substrates which will include but are not limited to monosaccharides such as glucose, oligosaccharides such as lactose or sucrose, polysaccharides such as starch or cellulose and unpurified mixtures from a renewable feedstocks such as cheese whey permeate, cornsteep liquor, sugar beet molasses, and barley malt. While it is contemplated that the source of carbon utilized in the present invention may encompass a wide variety of carbon containing substrates and will only be limited by the choice of organism, the preferred carbon substrates include glucose, fructose and sucrose and mixtures thereof. By using mixtures of glucose and fructose in combination with the altered bacterial strains described herein, uncoupling of the oxidative pathways from the catabolic pathways allows the use of glucose for improved yield and conversion to the desired ASA intermediate while utilizing the fructose to satisfy the metabolic requirements of the host cells. Fermentation media must also contain suitable minerals, salts, vitamins, cofactors and buffers suitable for the growth or the cultures and promotion of the enzymatic pathway necessary for ascorbic acid intermediate production.

### Batch and continuous fermentations:

The present invention may employ a batch fermentation process, a modified batch fermentation process, called Fed-batch or a continuous fermentation process. A classical batch fermentation is a closed system where the composition of the media is set at the beginning of the fermentation and not subject to artificial alterations during the fermentation. At the beginning of the fermentation the media is inoculated with the desired bacterial organism or organisms and fermentation is permitted to occur adding nothing to the system. Typically, however, a "batch" fermentation is batch with respect to the addition of carbon source and attempts are often made at controlling factors such as pH and oxygen concentration. In batch systems the metabolite and biomass compositions of the system change constantly up to the time the fermentation is stopped. Within batch cultures, cells moderate through a static lag phase to a high growth log phase and finally to a stationary phase where growth rate is diminished or halted. If untreated, cells in the stationary phase will eventually die. Cells in log phase generally are responsible for the bulk of production of desired product or intermediate.

A variation on the standard batch system is the Fed-Batch system. Fed-Batch fermentation processes are also suitable in the present invention and comprise a typical batch system with the exception that the substrate is added in increments as the fermentation progresses. Fed-Batch systems are useful when catabolite repression is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂. Batch and Fed-Batch fermentations are common and well known in the art and examples may be found in T.D. Brock in BIOTECHNOLOGY: A TEXTBOOK OF INDUSTRIAL MICROBIOLOGY, Second Edition (1989) Sinauer Associates, Inc. Sunderland, MA.

In one embodiment, the concentration of the carbon substrate in the feed solution is from about 55% to about 75% on a weight/weight basis. Preferably, the concentration is from about 60 to about 70% on a weight/weight basis. Most preferably the concentration used is 60% to 67% glucose.

Continuous fermentation is an open system where a defined fermentation media is added continuously to a bioreactor and simultaneously an equal amount of conditioned media is removed for processing. Continuous fermentation generally maintains the cultures at a constant high density where cells are primarily in log phase growth. Continuous fermentation allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Continuous systems strive to maintain steady state growth conditions and thus the cell loss due to media being drawn off must be balanced against the cell growth rate in the fermentation. Methods of modulating nutrients and growth factors for continuous fermentation processes as well as techniques for maximizing the rate of product formation are well known in the art of industrial microbiology and a variety of methods are detailed by Brock, *supra.*

### Increased yield of desired products from the ASA pathway.

In addition to increased accumulation of 2-KDG in altered bacterial host cells according to the invention, further embodiments may result in the uncoupling of the catabolic pathway from the ASA oxidative pathway to increase the availability of gluconate for 2-KDG production.

As shown in Figure 2, gluconate can be transported across the inner cell membrane to the cytoplasm. Gluconate may then be phosphorylated, for example to gluconate-6- phosphate and made available for catabolic metabolism in the pentose pathway. Additionally, gluconate may be enzymatically reduced to 5-KDG or 2-KDG in the cytoplasm. Inactivation or modification of the levels of a gluconate transporter by inactivation of the nucleic acid encoding the same, may result in an increased amount of gluconate available for the oxidative ASA production pathway. Additionally inactivation of a gluconokinase gene may result in a decrease of gluconate phosphorylation further increasing the amount of gluconate available for the oxidative ASA production pathway and the production of erythorbic acid.

The manner and method of carrying out the present invention may be more fully understood by those of skill in the art by reference to the following examples, which examples are not intended in any manner to limit the scope of the present invention or of the claims directed thereto. All references and patent publications referred to herein are hereby incorporated by reference.

### 6. EXAMPLES

### Example 1

### Inactivation of the orfs 2418 - 2420 encoding 2-Keto-D-Gluconate Dehydrogenase (2-KDGDH) Activity in P. citrea:

### A. Cloning of the KDGDH operon:

Strain 139-2a/Ps- was used for cloning the 2-KDGDH operon. This strain is a derivative of strain 139-2a having ATCC accession number 39140 wherein the cryptic plasmid (pS) is removed by the methods disclosed in WO 98/59054. Reference is also made to Truesdell et al., (1991) J. Bacteriol. 173:6651 - 6656.

Using two PCR primers; KDGF1 and KDGR1 a 2.8-kb DNA fragment encompassing the 2-KDGDH operon from the chromosome of *P. citrea* 139-2a/Ps- strain was amplified using standard techniques (Sambrook et al., MOLECULAR CLONING: A LABORATORY MANUAL, Cold Spring Harbor Laboratory Press (1989) 2nd Ed.).

| | | | |
|---|---|---|---|
| KDGF1 | 5' AGTTAGCCGCTCATTTCCTG | 3' | (SEQ ID NO. 7) |
| KDGR1 | 5' AGCCGCCTGGTTTTTAC | 3' | (SEQ ID NO. 8) |

The DNA fragment was cloned into the pZeroBlunt vector which has a lac promoter (IacP) (Invitrogen, Carlsbad CA) using *E. coli* TOP10 cells as a host. This resulted in plasmid pKDG2 (6.32-kb). On LA+Kan50 plates, (LB media solidified with 1.5% agar plus 50ppm kanamycin) three Kan^{R} transformants were obtained. When checked by digesting with appropriate restriction enzymes (*Eco*RI, *Sca*I + *Spe*I*, Sal*I *+ Spe*I*),* all three transformants were found to have inserts and transcriptional directions in all of them were opposite to the orientation of the IacP.

### B. Construction of the knockout plasmid used to delete the 2-KDGDH operon from the P. citrea chromosome:

In general, the strategy used to inactivate genes by homologous recombination with a plasmid has been delineated before and reference is made to Miller et al., (1988) J. Bacteriol. 170:2575 - 2583. This general approach was used to inactivate the 2-KDGDH operon.

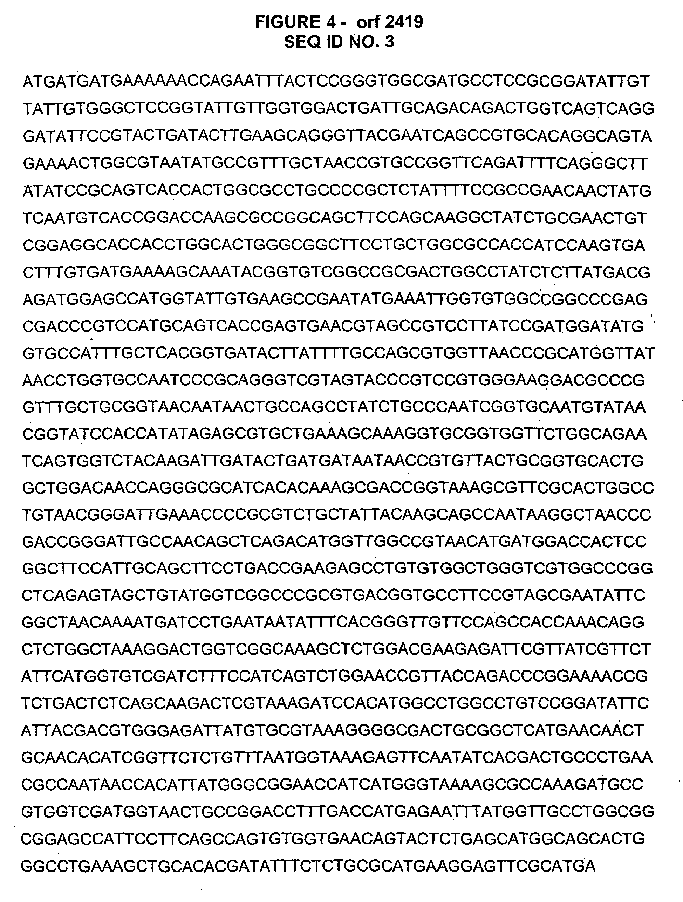

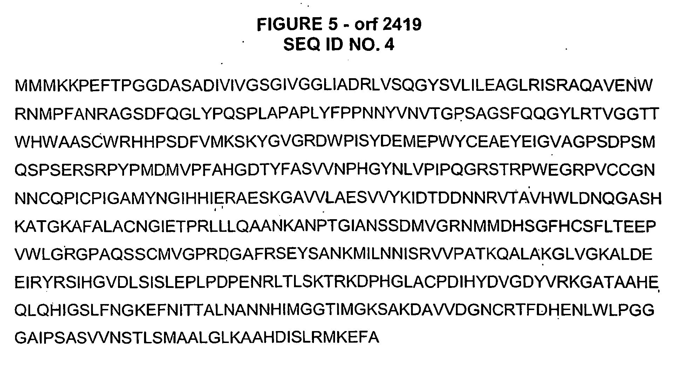

The pKDG2 plasmid obtained according to example 1A above, was digested with Hpal+Scal enzymes to eliminate a 0.993-kb region from the middle to C-terminus of the B subunit (2-KDGDH-B). The plasmid was then inserted with a cat cassette (1.080-kb) flanked by two IoxP sites (Palmeros et al., (2000) Gene 247:255 - 264) resulting in plasmid pKDGCat1 (6.41-kb; cat runs opposite to KDGDH operon). This plasmid was verified by digestion with Notl, SacI and Xbal enzymes. The 1.5-kb Aatll+Spel fragment containing the CoIE1 Ori region was removed from plasmid pKDGCat1, and then ligated with the 502-bp AatII+SpeI DNA fragment that contains the minimal R6K origin of replication (ori) region. The R6K ori DNA was obtained by PCR using plasmid pGP704 (Miller et al., (1988) J. Bacteriol. 170:2575 - 2583) as PCR substrate with primers. Thus the final knockout plasmid pKDGCatR6 (5.37-kb) was obtained. *E*. *coli* PIR1 strain (Invitrogen, Carlsbad, CA) was transformed using the procedure described in Sambrook et al. MOLECULAR CLONING: A LABORATORY MANUAL, Cold Spring Harbor Laboratory Press (1989) 2nd Ed. In this final knockout construct, 960-bp and 840-bp regions of homology are available at the 5'- and 3'-ends of the KDGDH operon to allow homologous recombination in *P*. *citrea* chromosome. The regions of homologies are represented by the orf illustrated in Figures 4 and 5 for subunit B.

### C. Transformation into P. citrea strain to obtain altered strains:

After the final knockout plasmid pKDGcatR6 (5.37 kb) was verified with HindIII digestion, the plasmid was electroporated (Sambrook et al., MOLECULAR CLONING: A LABORATORY MANUAL, Cold Spring Harbor Laboratory Press (1989) 2nd Ed.) into P. citrea 139-2a/Ps- (pKD46) competent cells and selected for chloramphenicol-resistant (CmR) transformants on LA+Cm10 plates (LA is LB plus agar plates with 10ppm Cm) using well known techniques. To distinguish between single and double crossover recombination events, the CmR transformants were checked on LA+Kan3 plates for kanamycin sensitivity (KanS). Nine of the 13 CmR transformants were KanS, implying that they had undergone a double crossover recombination event that inactivated the 2-KDGDH operon. Four transformants, Nos. 4, 5, 8 and 9 were checked by PCR with both internal and external primers as described below.

### D. PCR Verification of the knockout strains:

For verifying the KDGDH operon deletion, two outside primers will amplify the same size band both in the wild type containing a functional KDGDH operon and putative mutants (altered strains) wherein the KDGDH operon was deleted. (Reference is made to example 1C above wherein 0.993-kb was exchanged with 1.08-kb cat-IoxP DNA).

Thus one outside primer with one cat-gene-specific primer was used to verify the recombination junctions of the putative mutants. With cat3+KDGR2 primers, all four transformants amplified the expected 1.14-kb band as compared to the unaltered strain wherein there was no amplification. With KDGF2+cat4 primers, the transformants amplified the expected 1.17-kb band. This result revealed that the four transformants had undergone a double crossover recombination event at the KDG locus as expected thereby inactivating the operon.

| | | | |
|---|---|---|---|
| KDGF2 | 5' GCGTCTCTGCCATTGCGTAGTTTC | 3' | (SEQ ID NO. 9) |
| KDGR2 | 5' GGGTGCGGATCGGTGTGGTTT | 3' | (SEQ ID NO. 10) |
| CAT3 | 5' AAAGTTGGAACCTCTTACGTGCCG | 3' | (SEQ ID NO. 11) |
| CAT4 | 5' CAACAGTACTGCGATGAGTGGCAG | 3' | (SEQ ID NO. 12) |

### E. Removal of the pKD46 plasmid:

Since the altered'strains still contained plasmid pKD46 plasmid (Datsenko and Wanner (2000) Proc. Natl. Acad. Sci. 97:6640 - 6645) they were cured of this plasmid as follows. Cells were grown in liquid medium without Carbenicillin (Carb) at 30°C for 3 passages (3 days) followed by plating and isolation of single colonies. All single colony isolates lost the pKD46 plasmid when tested for Carb sensitivity on LA+Carb200 plates (Datsenko and Wanner, *supra* and Palmeros et al. (2000) Gene 247:255 - 264). Furthermore, no plasmid was detected in any of the single colony isolates when plasmid DNAs were isolated using standard protocols (Sambrook et al., *supra*). Altered *Pantoea* cells that were cured of plasmid pKD46 were obtained and designated WKDG4.

### Example 2

### Fermentation Experiments with Pantoea citrea.

All reagents and materials used for the growth of bacterial cells were obtained from Diffco Laboratories (Detroit, Mich.), Aldrich Chemicals (Milwaukee, Wis.) or Sigma Chemical Company (St. Louis, Mo.) unless otherwise specified.

Seed Train: Culture vials which were stored in liquid nitrogen containing the indicated strain WKDG4 were thawed in air and 0.75 mL was added to a sterile 2-L Erlenmeyer flask containing 500 mL of seed medium. Flasks were incubated at 29 °C and 250 rpm for 12 hours. Transfer criteria is an OD₅₅₀ greater than 2.5.

Seed flask medium - A medium composition was made according to the following: KH₂PO₄(12.0 g/L); K₂HPO₄ (4.0 g/L); MgSO4·7H₂O (2.0 g/L); Difco Soytone (2.0 g/L); Sodium citrate (0.1 g/L); Fructose (5.0 g/L); (NH₄)₂SO₄(1.0 g/L); Nicotinic acid (0.02.g/L); FeCl₃·6H₂O (5 mL/L of a 0.4 g/L stock solution) and Trace salts (5 mL/L -of the following solution: 0.58 g/L ZnSO₄·7H₂O, 0.34 g/L MnSO₄·H₂O, 0.48 g/L Na₂MoO₄·2H₂O). The pH of the medium solution was adjusted to 7.0 ± 0.1 unit with 20% NaOH. The resulting medium solution was then filter sterilized with a 0.2 µ filter unit. The sterile medium was added to a previously autoclaved flask.

Production Fermentor - Additions to the reactor vessel prior to sterilization included: KH₂PO₄(3.5 g/L); MgSO₄·7H₂O (1.0 g/L); (NH₄)₂SO₄(0.92 g/L); Mono-sodium glutamate (15.0 g/L); ZnSO₄·7H₂O (5.79 mg/L); MnSO₄·H₂O (3.44 mg/L); Na₂MoO₄·2H₂O (4.70 mg/L); FeCl₃·6H₂O (2.20 mg/L); Choline chloride (0.112 g/L) and Mazu DF-204 (0.167 g/L) an antifoaming agent.

The above constituted media was sterilized at 121°C for 45 minutes. After tank sterilization, the following additions were made to the fermentation tank: Nicotinic acid (16.8 mg/L); Ca-pantothenate (3.36 mg/L); Glucose (25g/L) and Fructose (25 g/L).

The final volume after sterilization and addition of post-sterilization components was 6.0 L. The so prepared tank and medium were inoculated with the full entire contents from seed flasks prepared as described to give a volume of 6.5 L.

Growth conditions were at 29°C and pH 6.0. Agitation rate, back pressure, and air flow were adjusted as needed to keep dissolved oxygen above zero. When the sugars initially batched into the medium were exhausted, a fed-batch process as previously described herein was employed. Glucose and fructose were used as substrates during the batch process and just glucose was used as a substrate during fed-batch.

As observed in Table 1 below, for a representative fermentation run, the production of 2-KDG obtained after a 30 hour time course with strain WKDG4 under fed-batch fermentation was significant compared with the isogenic wild type strain (139-2a/Ps-) which only makes 2-KDG transiently before it is further converted to 2,5-DKG.

**Table 1 - Fermentation Yield**

| Strain | 2-KDG conc (g/L) | 2-KDG Yield (wt %) | Productivity (g/L/hr) |
|---|---|---|---|
| 139-2a/Ps- | 0 | 0 | 0 |
| WKDG4 | 300 | 89 | 11.4 |

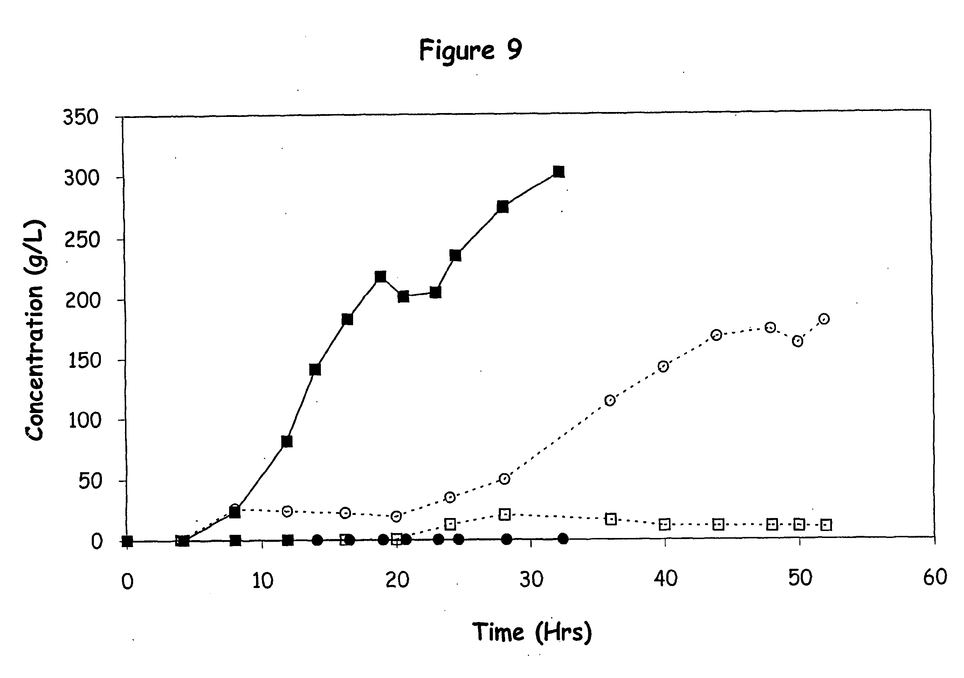

The results of another representative fermentation run are illustrated in Figure 9, wherein the altered strain, WKDG4 and the unaltered wild-type strain, 139-2a/Ps- are grown as described above. As observed from the figure, strain 139-2a/Ps- produces mostly 2,5-DKG, and reaches a maximum at 180 g/L. 2-KDG is accumulated transiently to a high of only 20 g/L and at the end of the run 2-KDG was only accumulated to a level of 10 g/L. In contrast, the altered strain (WKDG4) has a very different profile. 2,5-DKG was not detected and 2-KDG accumulated to a level as high as 302 g/L at the end of the run.

## Claims

1. A method for increasing the accumulation of 2-keto-D-gluconic acid (2-KDG) in a *Pantoea* host cell comprising:
a) inactivating in a *Pantoea* host cell, which is capable of producing 2,5-diketogluconate (2,5-DKG) from the enzymatic conversion of 2-KDG in the presence of a carbon source, at least one endogenous gene necessary for 2-keto-D-gluconate dehydrogenase (2-KDGDH) activity, wherein the at least one endogenous gene encodes (i) a dehydrogenase protein having at least 95% sequence identity to SEQ ID NO: 4, (ii) a cytochrome C protein having at least 95% sequence identity to SEQ ID NO 6, or (iii) a protein having at least 95% sequence identity to SEQ ID NO: 2, and wherein inactivating the at least one endogenous gene comprises inserting by homologous recombination an inactivation cassette comprising an antibiotic resistance marker to obtain an altered *Pantoea* cell; and
b) culturing the altered *Pantoea* cell under suitable culture conditions to produce 2-KDG to allow 2-KDG to accumulate in the *Pantoea* host cell.

2. The method according to claim 1, further comprising the step of recovering the 2-KDG.

3. The method according to claim 1, further comprising the step of converting the 2-KDG into erythorbic acid.

4. The method according to claim 4, wherein the *Pantoea* host cell is a *Pantoea citrea* host cell.

5. The method of claim 5, wherein the *Pantoea citrea* host cell is obtainable from ATCC Accession No: 39140

6. The method according to claim 1, wherein the at least one endogenous gene encodes a protein having dehydrogenase activity which has at least 97% sequence identity to SEQ ID NO: 2, 4 or 6.

7. The method according to claim 1, wherein the protein having dehydrogenase activity has the sequence of SEQ ID NO: 4.

## Patentansprüche

1. Verfahren zur Steigerung der Akkumulation von 2-Keto-D-gluconsäure (2-KDG) in einer *Pantoea*-Wirtszelle, umfassend:
a) Inaktivierung in einer *Pantoea*-Wirtszelle, die zur Produktion von 2,5-Diketogluconat (2,5-DKG) aus der enzymatischen Umwandlung von 2-KDG bei Vorliegen einer Kohlenstoffquelle fähig ist, mindestens eines endogenen Gens, das für die 2-Keto-D-gluconatdehydrogenase-Aktivität (2-KDGDH-Aktivität) notwendig ist, wobei das mindestens eine endogene Gen für (i) ein Dehydrogenase-Protein mit einer mindestens 95%igen Sequenzidentität mit SEQ ID NO: 4, (ii) ein Cytochrom-c-Protein mit einer mindestens 95%igen Sequenzidentität mit SEQ ID NO: 6 oder (iii) ein Protein mit einer mindestens 95%igen Sequenzidentität mit SEQ ID NO: 2 kodiert und wobei die Inaktivierung des mindestens einen endogenen Gens das Insertieren durch homologe Rekombination einer Inaktivierungskassette umfasst, die einen Antibiotika-Resistenz-Marker zum Erhalt einer veränderten *Pantoea*-Zelle umfasst; und
b) Kultivierung der veränderten *Pantoea*-Zelle unter geeigneten Kulturbedingungen zur Produktion von 2-KDG, um die Akkumulation von 2-KDG in der *Pantoea*-Wirtszelle zuzulassen.

2. Verfahren nach Anspruch 1, ferner umfassend den Schritt zur Rückgewinnung der 2-KDG.

3. Verfahren nach Anspruch 1, ferner umfassend den Schritt zur Umwandlung der 2-KDG in Erythorbinsäure.

4. Verfahren nach Anspruch 4, wobei die Pantoea-Wirtszelle eine *Pantoea citrea*-Wirtszelle ist.

5. Verfahren nach Anspruch 5, wobei die *Pantoea citrea*-Wirtszelle über die ATCC-Zugangsnummer: 39140 erhältlich ist.

6. Verfahren nach Anspruch 1, wobei das mindestens eine endogene Gen für ein Protein mit Dehydrogenase-Aktivität kodiert, das eine mindestens 97%ige Sequenzidentität mit SEQ ID NO: 2, 4 oder 6 aufweist.

7. Verfahren nach Anspruch 1, wobei das Protein mit Dehydrogenase-Aktivität die Sequenz von SEQ ID NO : 4 aufweist.

## Revendications

1. Procédé d'augmentation de l'accumulation d'acide 2-céto-D-gluconique (2-KDG) dans une cellule hôte *Pantoea,* comprenant:
a) inactiver dans une cellule hôte *Pantoea,* qui est capable de produire du 2,5-dicétogluconate (2,5-KDG) à partir de la conversion enzymatique de 2-KDG en présence d'une source de carbone, au moins un gène endogène nécessaire à l'activité 2-céto-D-gluconate déshydrogénase (2-KDGDH), où le au moins un gène endogène code (i) une protéine déshydrogénase ayant au moins 95 % d'identité de séquence avec la SEQ ID NO: 4, (ii) une protéine cytochrome C ayant au moins 95 % d'identité de séquence avec la SEQ ID NO: 6 ou (iii) une protéine ayant au moins 95 % d'identité de séquence avec la SEQ ID NO: 2, et où inactiver le au moins un gène endogène comprend insérer par recombinaison homologue une cassette d'inactivation comprenant un marqueur de résistance aux antibiotiques, pour obtenir une cellule *Pantoea* altérée; et
b) cultiver la cellule *Pantoea* altérée dans des conditions de culture appropriées pour produire du 2-KDG afin de permettre au 2-KDG de s'accumuler dans la cellule hôte *Pantoea.*

2. Procédé selon la revendication 1, comprenant en outre l'étape consistant à recueillir le 2-KDG.

3. Procédé selon la revendication 1, comprenant en outre l'étape consistant à convertir le 2-KDG en acide érythorbique.

4. Procédé selon la revendication 4, dans lequel la cellule hôte *Pantoea* est une cellule hôte *Pantoea citrea.*

5. Procédé selon la revendication 5, dans lequel la cellule hôte *Pantoea citrea* peut être obtenue sous le no d'accès ATCC 39140.

6. Procédé selon la revendication 1, dans lequel le au moins un gène endogène code une protéine ayant une activité déshydrogénase qui a au moins 97 % d'identité de séquence avec la SEQ ID NO: 2, 4 ou 6.

7. Procédé selon la revendication 1, dans lequel la protéine ayant l'activité déshydrogénase a la séquence de la SEQ ID NO: 4.
